# EUROPÄISCHE PATENTSCHRIFT

(11) **EP 0 419 419 B1**
(45) Veröffentlichungstag und Bekanntmachung des Hinweises auf die Patenterteilung: **24.11.1993**
(21) Anmeldenummer: 90810716.2
(22) Anmeldetag: 19.09.1990
(51) Int. Cl.: C07C 41/03, B01J 19/24, B01J 19/26

(54) **Verfahren zur sicheren und umweltschonenden Herstellung hochreiner Alkylenoxid-Addukte**
Process for the safe and pollution-free preparation of highly pure alkylene oxide adducts
Procédé de préparation des produits d'addition d'oxydes alkylènes d'une manière sûre et non polluante

(30) Priorität: 22.09.1989 CH 3586/89
(43) Veröffentlichungstag der Anmeldung: 27.03.1991
(73) Patentinhaber: BUSS AG, BASEL, CH-4133 Pratteln 1 (CH)
(72) Erfinder: Leuteritz, Günter M., Dr., CH-4133 Pratteln (CH)
(74) Vertreter: Zimmermann, Hans, Dr.

(56) Entgegenhaltungen:
- AT-A- 287 890
- DE-A- 2 130 519
- US-A- 2 586 767

## Beschreibung

Die Erfindung betrifft ein Verfahren zur Herstellung von Alkylenoxid-Addukten organischer Verbindungen, die wenigstens ein reaktives Wasserstoffatom enthalten, welches über ein Sauerstoff-, Stickstoff- oder Schwefelatom mit einem organischen Molekülrest verbunden ist.

Die eingesetzten organischen Verbindungen sind unter Reaktionsbedingungen flüssig; dagegen sind die eingesetzten Alkylenoxide unter den Reaktionsbedingungen gasförmig. Als Alkylenoxide kommen insbesondere Ethylenoxid, Propylenoxid, Butylenoxid und andere unter 200°C siedende Alkylenoxide in Frage.

Alkylenoxide sind hochreaktive Verbindungen, die sich in stark exothermer Reaktion an reaktive organische Verbindungen anlagern. Deshalb ist es wichtig, für eine sichere und ausreichende Abfuhr der Reaktionswärme zu sorgen. Besonders erschwerend kommt aber hinzu, dass Alkylenoxide mit Luft explosionsartig reagieren können und sich auch selbst ohne Lufteinwirkung, z.B. oberhalb von 450 C°/10 bar, explosionsartig zersetzen.

Aus diesem Grunde ist es seit jeher ein Bestreben, Alkylenoxide unter extremem Luftausschluss, und zwar in möglichst geringen kontrollierten Mengen, zur Reaktion zu bringen.

Dem Fachmann ist unter anderem aus der Uebersicht von Winnacker/Küchler, Band 7, Organische Technologie III, 4. Auflage, C. Hanser Verlag München Wien 1986, Seiten 129 bis 131, bekannt, die Umsetzung zwischen den oben beschriebenen Ausgangsverbindungen in einem Rührkessel mit oder ohne Inertgas-Abdeckung unter Verwendung von alkalischen oder sauren Katalysatoren bei Temperaturen oberhalb von 100 C° und unterhalb von 200 C° durchzuführen.

Des weiteren ist aus der US-Patentschrift 2586767 bekannt, die Reaktion unter sonst gleichen Bedingungen in einem Schleifenreaktor durchzuführen. Bei diesem Verfahren wird die flüssige Ausgangskomponente mittels einer Umwälzpumpe über einen nachgeschalteten Wärmeaustauscher durch eine Sprühdüse in feinverteilter Form in die gasförmige Alkylenoxid-Atmosphäre des Reaktors gesprüht. Die gegenüber dem Rührkessel deutlich verbesserte Wärmeabfuhr erlaubt erheblich verkürzte Reaktionszeiten bei Umsetzungsraten in der Grössenordnung von z.B. 400 kg Ethylenoxid/m³ Reaktorvolumen und Stunde.

In Abwandlung des Verfahrens gemäss der US-Patentschrift 2586767 wird in der Abhandlung "Evolution of Ethoxylation Plants for Nonionic Surfactants Production" (P. Straneo, C. Maffezoni und A. Marchegiano, Pressindustria Engineering and Plants S.p.A., Biassono, Italien, vorgetragen auf dem Welt-Tenside-Kongress, 6.5. bis 10.5.1984 in München) ein Verfahren vorgestellt, bei dem die flüssige Ausgangskomponente ebenfalls in die gasförmige Alkylenoxid-Atmosphäre innerhalb eines spezifischen Kontaktors gesprüht wird. Dieses Verfahren ist nachteilig gekennzeichnet durch eine nicht gekühlte Gasphase, durch die Ausbildung eines weiteren, nicht mit der Kontaktor-Gasphase in Verbindung stehenden Gasraumes und den Verzicht auf die Zwangsumwälzung der Flüssigphase innerhalb des Kontaktors als Folge der schwerkraftbestimmten Förderung der Flüssigphase vom Kontaktor zum Reaktor.

Allen obigen Verfahren ist zu eigen, dass das flüssige Ausgangsprodukt in eine oder mehrere statische, nicht gekühlte und ungenügend durchmischte Gasphasen aus Alkylenoxid mit oder ohne Inertgasanteil eingebracht wird.

Des weiteren wird in dem Artikel "Der Buss-Schleifenreaktor in der Oel- und Fetthärtungsindustrie" (R.F. Duveen und G. Leuteritz, Buss AG Basel) in Fette, Seifen, Anstrichmittel 84, 511-515, 1. Sonderheft 1982) ein Hydrierverfahren beschrieben, in dem eine Strahlsauger-Mischdüse grosse Phasenaustauschflächen zwischen Reaktionsgas und Flüssigkeit erzeugt, wobei zwangsläufig das gasförmig zugeführte Reaktionsgas mit dem gegebenenfalls anwesenden Inertgas ständig intensiv gemischt und umgewälzt wird.

Dieses Verfahren für die Umsetzung von Gasen mit Flüssigkeiten hat bezüglich der vorgeschlagenen Anwendung auf Alkoxylierungen sowohl hinsichtlich der Produktqualität, der Raum/Zeit-Ausbeute wie auch der Betriebssicherheit bereits Vorteile gegenüber den anderen, oben genannten Verfahren ohne einen über eine Strahlsauger-Mischdüse aneinander gekoppelten Gas- und Flüssigkeits-Kreislauf. So wird bei erzielbarer hoher Raum/Zeit-Ausbeute mit Werten bis zu 1500 kg Ethylenoxid und mehr je m³-Reaktorvolumen und Stunde auf Grund der sehr starken Umwälzung der Flüssigphase wie auch der Gasphase bei sehr exakter Temperaturführung eine deutlich verbesserte Produktqualität erzielt.

Des weiteren wird durch die stetige intensive Homogenisierung der einheitlichen, in sich geschlossenen Gas- wie auch Flüssigphase in Folge der starken Umwälzung die Sicherheit dieses Verfahrens durch Vermeidung von lokalen Inhomogenitäten bezüglich Temperatur, Alkylenoxid- und Katalysatorkonzentration ganz erheblich verbessert.

Ferner wird in der Patentschrift AT 287 890 beschrieben, wie bei einem anderen Hydrierverfahren für organische Verbindungen durch eine drosselbare und geregelte Wasserstoffzufuhr zur Mischdüse der Ansaugdruck der Mischdüse unabhängig vom Gasdruck über der Flüssigkeit im Oberteil des Autoklaven eingestellt werden kann und damit sowohl die Reaktionsgeschwindigkeit als auch die Selektivität bestimmter Reaktionen beeinflusst werden können.

Im Hinblick auf die Freiheit von toxischen Verunreinigungen der Endprodukte sind die Forderungen des Marktes in der letzten Zeit besonders drastisch gestiegen. In diesem Zusammenhang ist die Herabsetzung des Restgehaltes an nicht umgesetzten Alkylenoxiden wie auch an den sich aus den Alkylenoxiden während der Reaktion durch Anlagerungs- und Umlagerungsreaktionen bildenden Nebenprodukten zu nennen. Bei der Herstellung von Ethylenoxid-Addukten sind diese insbesondere Ethylenoxid selbst, Dioxan und Dioxolan.

Die Einwirkung eines sauren Milieus, sei es z.B. ein saurer Katalysator oder auch ein saurer Reaktionspartner selbst, wie z.B. eine Fettsäure, begünstigt in extremem Masse die Bildung der genannten Nebenprodukte.

Um die heute in vielen Ländern geltenden gesetzlich vorgeschriebenen Bestimmungen zum Schutze des Verbrauchers vor gesundheitsschädlichen Stoffen zu erfüllen, muss der Gehalt an Ethylenoxid unterhalb von 1 ppm und der Gehalt an Dioxan unterhalb von 10 ppm liegen.

Diese niedrigen Ethylenoxid- und Dioxan-Werte konnten bisher üblicherweise nur durch aufwendige Reinigungsverfahren in gesonderten Behandlungsanlagen mittels zusätzlicher Verfahrensschritte erreicht werden. Bezüglich der Vermeidung der Bildung von Dioxan kann keines der Verfahren des Standes der Technik die heute geforderten Grenzwerte im eigentlichen Reaktionsschritt erreichen. In allen Fällen ist eine zeit- und kostenaufwendige zusätzliche Nachbehandlung überwiegend in einer gesonderten Apparatur, zum Austreiben der toxischen Spuren mittels einer Trägergas-Behandlung mit z.B. Wasserdampf und/oder Stickstoff unter Vakuum bei hoben Temperaturen notwendig.

Nachteilig ist darüber hinaus, dass die bei der Trägergas-Behandlung entstehenden erheblichen Abgasmengen heute ebenso wie die bei der Herstellung anfallenden toxischen Abgasmengen in Abgasreinigungsanlagen nach dem Wäscher- bzw. Adsorptionsprinzip oder durch Verbrennung aufwendig gereinigt werden müssen.

Die zur Entfernung der toxischen Verunreinigungen angewandten Verfahren mittels Trägergas-Behandlung oder auch Adsorption an Molekularsieben, wie in der DOS 3740695 vorgeschlagen, können gleichzeitig, nötigenfalls unter geänderten Bedingungen auch zur deutlichen Verbesserung der Gebrauchseigenschaften von Alkylenoxid-Addukten im Hinblick auf Geruch und Farbe beitragen. Wie überaus wichtig die verlangte Verbesserung der Gebrauchseigenschaften der Produkte selbst wie auch die Beständigkeit der Produkte im Hinblick auf weitere Umsetzungen ist, geht auch aus der DOS 3604035 deutlich hervor, in welcher als Lösung der Zusatz von bestimmten Additiven vorgeschlagen wird.

Der Erfindung liegt also die Aufgabe zugrunde, hochreine Produkte gemäss heutigen und zukünftig zu erwartenden Reinheitsanforderungen durch ein sicheres und umweltschonendes Herstellungsverfahren in einer einzigen Stufe in einer Anlage und dadurch besonders wirtschaftlich herzustellen.

Ueberraschenderweise gelingt dies dadurch, dass man die Reaktion in dem bereits erwähnten Schleifenreaktor mit integrierter Strahlsauger-Mischdüse über einen gekoppelten Gas- und Flüssigkeits-Kreislauf durchführt, wobei man die Gasphase aus Reaktionsgas allein oder mit Zumischung von Inertgas innerhalb des geschlossenen Gaskreislaufes während der Anlagerungsreaktion auf Temperaturen unterhalb der Temperatur der Flüssigphase hält. Der Temperaturunterschied der beiden Phasen sollte mindestens um 1 °C liegen. Das Reaktionsgas ist das jeweilige Alkylenoxid, während als Inertgas jedes beliebige Gas eingesetzt werden kann, welches bei den Reaktionsbedingungen nicht mit sich selbst oder den Reaktionspartnern reagiert. Vorzugsweise wird Stickstoff oder Kohlendioxid als Inertgas eingesetzt.

Auf Grund zahlreicher Untersuchungen über die Wirkung der Katalysatoren, insbesondere der sauren Typen, musste der Fachmann bisher annehmen, dass die Bildung von Nebenprodukten der Alkylenoxide, insbesondere des Dioxans, im wesentlichen in der durch Temperatur und Katalysator aktivierten Flüssigphase stattfindet, wie insbesondere aus DOS 2300248 hervorgeht.

Die Herabsetzung der Temperatur der Gasphase erfolgt mit Vorteil durch Zugabe von flüssigem Ethylenoxid in den Gasraum des Reaktors. Diese Zugabe kann in beliebiger Art und Weise, vorteilhaft durch Feinverteilung, z.B. über einen Düsenring, erfolgen.

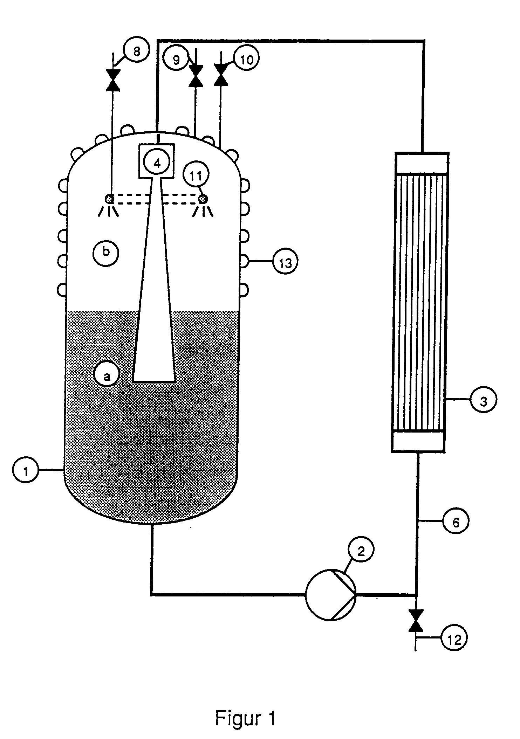

Zusätzlich kann die Kühlung der Gasphase durch Nutzung der Anlagenwandungen als Kühlfläche intensiviert werden. Dies geschieht vorteilhafterweise bei innen angeordneter Strahlsauger-Mischdüse durch Kühlung der Reaktorwandungen (siehe Fig. 1).

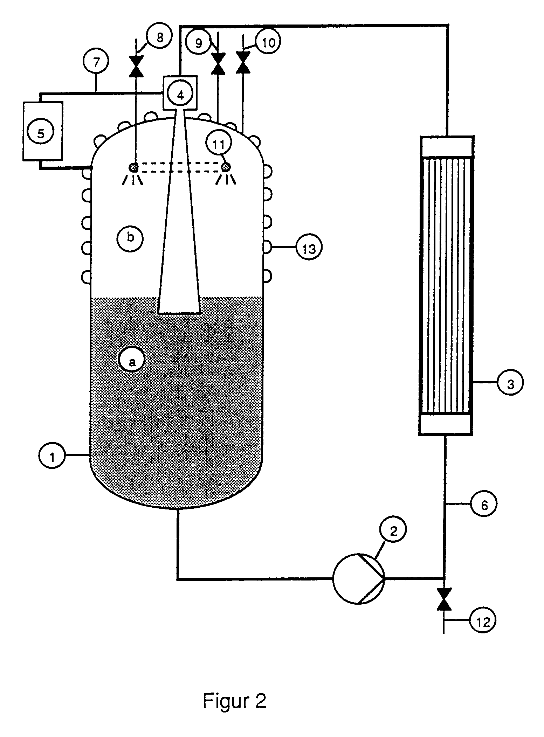

Bei aussen angeordneter Strahlsauger-Mischdüse können sowohl die Verbindungsleitung zwischen Reaktorraum und Strahlsauger-Mischdüse wie auch gegebenenfalls eingebaute Wärmeaustauscher zusätzlich als Kühlfläche genutzt werden (siehe Fig. 2).

Das Verfahren wird mit Vorteil in einer Anlage gemäss beiliegender Figur 1 oder Figur 2 durchgeführt. Es haben darin die Ziffern folgende Bedeutung:
- 1:: Reaktor
- 2:: Umwälzpumpe
- 3:: Wärmeaustauscher der Flüssigphase
- 4:: Strahlsauger-Mischdüse
- 5:: Wärmeaustauscher der Gasphase
- 6:: Umwälzleitung der Flüssigphase
- 7:: Umwälzleitung der Gasphase
- 8:: Zufuhrleitung für flüssiges Alkylenoxid
- 9:: Entleerungsleitung der Gasphase
- 10:: Zufuhrleitung des Inertgases
- 11:: Düsenring für flüssiges Alkylenoxid
- 12:: Zufuhr- und Entleerungsleitung der Flüssigphase
- 13:: Gekühlte Reaktorwandung

a) Flüssigphase
b) Gasphase

Es hat sich als besonders vorteilhaft für die Produktqualität erwiesen, wenn das Kühlen der Gasphase mit einer starken Durchmischung von Gas- und Flüssigphase einhergeht; dazu ist es erforderlich, dass der Treibstrahl des Strahlsaugers eine Austrittsgeschwindigkeit von mindestens 10 m/sec,vorteilhaft mehr als 20 m/sec aufweist und die Umwälzung der Flüssigphase so bemessen ist, dass der Reaktorinhalt zu jedem Zeitpunkt während der Reaktion mindestens 20mal pro Stunde, vorzugsweise mehr als 30mal pro Stunde umgewälzt wird.

Des weiteren hat sich als vorteilhaft erwiesen, den Partialdruck des Inertgases von Reaktionsbeginn an mindestens auf einen Wert oberhalb von 80% des Partialdruckes des Alkylenoxides einzustellen, vorzugsweise auf einen Inertgas-Partialdruck von 1 bis 25 bar, insbesondere aber auf einen Druck im Bereich von 3 bis 7 bar.

Durch diese Massnahmen, einzeln oder gegebenenfalls auch nebeneinander, gelingt es entgegen den Erwartungen die Bildung von toxischen Nebenprodukten der Alkylenoxide selbst und anderer die Gebrauchseigenschaften verschlechternden Nebenprodukte praktisch völlig zu unterdrücken.

Im Vergleich zu den Verfahren des Standes der Technik, bei welchen bei der Ethoxylierung in den Rührkesselanlagen sich üblicherweise Ethylenoxidwerte deutlich über 100 ppm und Dioxanwerte im Bereich von 500 ppm bis 2000 ppm einstellen und in den Schleifenreaktoren je nach Verfahrensbedingungen und Produktart häufig Ethylenoxidwerte über 10 ppm und Dioxanwerte zwischen 100 ppm und 1000 ppm unvermeidbar sind, werden nach dem erfindungsgemässen Verfahren Ethylenoxidgehalte unter 1 ppm und Dioxangehalte unter 10 ppm erreicht.

Darüber hinaus führt die niedrige Temperatur/Zeit-Belastung der Alkylenoxid-Gasphase zu besonders nebenproduktfreien, hellen und geruchsneutralen Addukten.

Weitere Vorteile des erfindungsgemässen Verfahrens sind die Vermeidung von dioxanhaltigen grossen Abgasmengen bei gleichzeitiger Erhöhung der Ausbeute.

Darüber hinaus bedeutet das erfindungsgemässe Verfahren einen erheblichen Gewinn an verfahrenstechnischer Sicherheit, da die (durch autokatalytische Zersetzung gefährdete Alkylenoxid-Gasphase zusätzlich zu der Zwangshomogenisierung durch die Gasumwälzung gleichzeitig auch noch gekühlt wird und damit die Möglichkeit einer gefährlichen Zersetzung ausgeschlossen wird.

Das erfindungsgemässe Verfahren soll anhand der nachfolgenden Beispiele näher erläutert werden.

### Vergleichsbeispiel 1

Herstellung von Polyoxyethylen-20-laurylether im Rührautoklaven

In einem Rührautoklaven von 50 l Nutzinhalt, der mit Einleitungsrohr und Heiz- und Kühlvorrichtung versehen ist, werden 9,3 kg Fettalkohol (Lorol 1216, Hersteller: Henkel KGaA, Düsseldorf BRD; Zusammensetzung: ca. 62% Laurylalkohol, 23% Myristylalkohol) eingefüllt und auf 100 C° erhitzt. Hierauf werden 0,04 kg Natriumhydroxid-Pulver (98%ig) als Katalysator zugegeben und intensiv vermischt. Der Autoklav wird verschlossen und das Gemisch unter Vakuum getrocknet. Das Vakuum wird dann mit Stickstoff aufgehoben und noch zweimal evakuiert und das Vakuum jeweils mit Stickstoff aufgehoben. Danach wird das Gemisch unter Rühren auf 165 C° erhitzt.

Bei Erreichen dieser Temperatur wird durch das Einleitungsrohr, welches in die Flüssigkeit eintaucht, unter Einhaltung eines maximalen Druckes im Autoklaven von 4 bar 0,15 kg flüssiges Ethylenoxid zugegeben. Nach 16 Minuten ist durch einen leichten Anstieg der Temperatur auf 175 C° und gleichzeitigen Abfall des Reaktordruckes der Beginn der Reaktion erkennbar.

Zu diesem Zeitpunkt wird unter stetigem Rühren und gleichzeitigem Kühlen unter Beachtung des maximalen Reaktordruckes die Zugabe von 42 kg flüssigem Ethylenoxid mit einer kontinuierlichen Zugaberate von ca. 14 kg/Std. begonnen. Der Druck im Autoklaven stellt sich zu Beginn der Zugabephase auf 3 bar ein und erreicht zum Ende der Ethylenoxidzugabe einen Wert von 4 bar. Nach Beendigung der Ethylenoxidzugabe nach ca. 3,5 Stunden wird unter Rühren eine halbe Stunde bei Reaktionstemperatur nachreagieren lassen. Der Druck im Reaktor fällt auf 2,5 bar.

Es wird eine Probe genommen und gaschromatographisch untersucht. Dieses Reaktionsprodukt, ein Laurylalkohol angelagert mit 20 Mol Ethylenoxid, enthielt 130 ppm Ethylenoxid und 600 ppm Dioxan.

### Vergleichsbeispiel 2

Herstellung von Polyoxyethylen-20-laurylether im Schleifenreaktor gemäss US 2,586,767

In einen Schleifenreaktorgemäss US 2,586,767, bestehend aus einem Reaktionsautoklaven mit 50 1-Nutzinhalt, einer Umwälzpumpe, einem Wärmeaustauscher und einer Sprüheinheit, werden dieselben Produkte in denselben Mengen wie im Vergleichsbeispiel 1 auf die dort angegebene Weise eingefüllt, erhitzt und getrocknet. Der Reaktor wird verschlossen, dreimal evakuiert und jeweils das Vakuum mit Stickstoff wieder aufgehoben. Die Temperatur wird unter Umwälzung auf 165 C° erhöht.

Bei Erreichen dieser Temperatur wird die Anlagerung mit der Zugabe von 300 g flüssigem Ethylenoxid innerhalb eines Zeitraumes von 10 Minuten unter Beachtung eines maximalen Autoklavendruckes von 4 bar begonnen. Der Beginn der Reaktion wird durch einen Anstieg der Temperatur um ca. 10 C° und gleichzeitigen Rückgang des Autoklavendruckes am Ende dieses Zugabezeitraumes nach 10 Minuten erkennbar.

Nach dem Start der Reaktion werden weitere 42 kg Ethylenoxid mit einer Zugaberate von 17 kg/Std. unter kontinuierlicher Abfuhr der Reaktionswärme durch Kühlung angelagert. Der Druck in der Anlage steigt von 3 bar in der ersten Phase der Reaktion auf 4 bar zum Ende der Ethylenoxid-Zugabe an. Innerhalb von 10 Minuten nach Beendigung der Ethylenoxid-Zugabe fällt der Druck in der Anlage auf 2 bar.

Zu diesem Zeitpunkt wurde eine Probe des Reaktionsproduktes entnommen und gaschromatographisch untersucht. Es wurde ein Gehalt von 12 ppm Ethylenoxid und 46 ppm Dioxan gefunden.

### Vergleichsbeispiel 3

Herstellung von Polyoxyethylen-20-laurylether im Schleifenreaktor gemäss US 2,586,767 ergänzt mit einer innen angeordneten Strahlsaugermischdüse.

Die Anlage gemäss Vergleichsbeispiel 2 wurde durch Austausch der Sprühdüse gegen eine Strahlsauger-Mischdüse geändert. Unter sonst gleichen Bedingungen und unter Verwendung der gleichen Einsatzmengen wurde der Versuch gemäss Vergleichsbeispiel 2 unter Verwendung von gasförmigem Ethylenoxid anstelle von flüssigem Ethylenoxid durchgeführt.

Der Druck in der Anlage stieg von einem Anfangswert zu Beginn der Reaktion von 2 bar auf einen Wert von 3,5 bar zum Ende der Ethylenoxid-Zugabe. Nach Beendigung der Ethylenoxid-Zugabe fiel der Druck innerhalb von 2 Minuten auf 2 bar zurück.

Nach weiteren 8 Minuten Umwälzung bei 165 C° wurde eine Probe entnommen und analysiert. Der Ethylenoxid-Gehalt lag unter 1 ppm, während der Gehalt an Dioxan 18 ppm betrug.

### Vergleichsbeispiel 4

Herstellung von Polyoxyethylen-20-stearaten im Rührautoklaven

In einem Rührautoklaven von 50 l Nutzinhalt, der mit Einleitungsrohr und Heiz- und Kühlvorrichtung versehen ist, werden 12 kg Stearinsäure (Edenor St1, Hersteller: Henkel KGaA, Düsseldorf BRD; Zusammensetzung: ca. 46% Palmitinsäure, 49% Stearinsäure) eingefüllt und auf 100°C erhitzt. Hierauf werden 0,05 kg Natriumhydroxid-Pulver (98%ig) als Katalysator zugegeben und intensiv vermischt. Der Autoklav wird verschlossen und das Gemisch unter Vakuum getrocknet. Das Vakuum wird dann mit Stickstoff aufgehoben und noch zweimal evakuiert und das Vakuum jeweils mit Stickstoff aufgehoben. Danach wird das Gemisch unter Rühren auf 165°C erhitzt.

Bei Erreichen dieser Temperatur wird durch das Einleitungsrohr, welches in die Flüssigkeit eintaucht, unter Einhaltung eines maximalen Druckes von 6 bar im Autoklaven so zügig wie möglich die gesamte Ethylenoxid-Menge von 38 kg in Teilmengen zugegeben.

Die Zugabe der ersten 8 kg Ethylenoxid benötigt ungefähr 3 Stunden, während die restlichen 30 kg Ethylenoxid in weiteren 4,5 Stunden zugegeben wurden. Nach Beendigung der Ethylenoxidzugabe nach ca. 7,5 Stunden wird unter Rühren eine halbe Stunde bei Reaktionstemperatur nachreagieren lassen. Der Druck im Reaktor fällt auf ca. 3 bar.

Es wird eine Probe genommen und gaschromatographisch untersucht. Dieses Reaktionsprodukt, eine Stearinsäure angelagert mit 20, Mol Ethylenoxid, enthielt 150 ppm Ethylenoxid und ca. 3000 ppm Dioxan.

### Vergleichsbeispiel 5

Herstellung eines Polyoxyethylen-20-stearates im Schleifenreaktor gemäss US 2 586 767

In einen Schleifenreaktor nach Vergleichsbeispiel 2 werden 12 kg Stearinsäure (Edenor St1 der Firma Henkel KGaA, Düsseldorf BRD; Zusammensetzung: 46% Palmitinsäure und 49% Stearinsäure) mit 0,05 kg NaOH-Pulver (98%ig) als Katalysator bei einer Temperatur von 100°C eingefüllt, vermischt und intensiv getrocknet. Der Reaktor wird verschlossen, dreimal evakuiert und jeweils das Vakuum mit Stickstoff wieder aufgehoben. Danach wird die Temperatur unter Umwälzung auf 165°C erhöht und der Reaktor gleichzeitig nochmals evakuiert.

Bei Erreichen dieser Temperatur wird die Reaktion mit der Zugabe von flüssigem Ethylenoxid bis zu einem Druckanstieg auf 5,8 bar gestartet. Die Zugabe von 8 kg Ethylenoxid benötigt ungefähr 1,5 Stunden. Zum Ende dieser Phase stellt sich der Reaktordruck auf 4,5 bar ein, die restlichen 30 kg Ethylenoxid werden sodann im Verlauf der nächsten 3 Stunden zugegeben. Der Reaktordruck betrug ca. 4,5 bar. Die Temperatur des Produktes stieg auf 175°C.

0,5 Stunden nach Beendigung der Ethylenoxid-Zufuhr wurde eine Probe entnommen und analysiert. Es wurde ein Restgehalt an Ethylenoxid von 95 ppm und ein Dioxan-Gehalt von ungefähr 1400 ppm bestimmt.

### Beispiel 1

Herstellung von Polyoxyethylen-20-laurylether im Schleifenreaktor mit aussen angeordneter Strahlsauger-Mischdüse und einem innen angeordneten Düsenring (Fig. 2).

Die Anlage gemäss Vergleichsbeispiel 3 wurde durch Verlegung der Strahlsauger-Mischdüse nach aussen und Einbau einer Rohrverbindung mit Wärmeaustauscher zwischen dem Gasraum des Reaktors und dem Ansaugstutzen der Strahlsauger-Mischdüse abgeändert. Zusätzlich wurde anstelle des einfachen Einleitungsstutzen ein Düsenring in den Kopfraum des Autoklaven zur Einspeisung von flüssigem Ethylenoxid eingebaut.

Unter sonst gleichen Bedingungen und mit den gleichen Stoffmengen wurde der Versuch gemäss Vergleichsbeispiel 3 wiederholt. Nur wurde in Abänderung des Versuches das Ethylenoxid nicht gasförmig, sondern flüssig zugegeben; Austrittsgeschwindigkeit des Treibstrahls 20 m/Sek. und 30 Flüssigkeitsumwälzungen (Endvolumen) pro Stunde. Der Reaktionsablauf war identisch mit dem Vergleichsbeispiel 3.

Die 10 Minuten nach Beendigung der Ethylenoxidzugabe gezogene Produktprobe enthielt einen Restgehalt an Ethylenoxid unter 1 ppm, während der Gehalt an Dioxan bei 5 ppm lag.

### Beispiel 2

Herstellung eines Polyoxyethylen-20-stearates im Schleifenreaktor (Fig. 1).

In einen Schleifenreaktor nach Figur 1 werden 12 kg Stearinsäure (Edenox ST1 der Firma Henkel KGaA, Düsseldorf BRD; Zusammensetzung: 46% Palmitinsäure und 49% Stearinsäure) mit 0,05 kg NaOH-Pulver (98%ig) als Katalysator mit einer Temperatur von 100 C° eingefüllt, vermischt und intensiv getrocknet. Der Reaktor wird verschlossen, dreimal evakuiert und jeweils das Vakuum mit Stickstoff wieder aufgehoben. Die Temperatur wird unter Umwälzung auf 165 C° erhöht.

Bei Erreichen dieser Temperatur wird die Reaktion mit der Zugabe von 1 kg flüssigem Ethylenoxid unter einem Druckanstieg auf 4,8 bar gestartet. Weitere 7 kg Ethylenoxid werden in den folgenden 20 Minuten zugegeben. In dieser Phase stellt sich der Reaktordruck auf 4,5 bar ein. Die restlichen 30 kg Ethylenoxid werden sodann im Verlauf der nächsten 30 Minuten zugegeben. Der Reaktordruck bewegte sich zwischen 4,5 und 4,0 bar. Die Temperatur des Produktes stieg auf 175 C°. Innerhalb einer Minute fiel der Druck nach Beendigung der Ethylenoxid-Zugabe auf 1,8 bar.

10 Minuten nach Beendigung der Ethylenoxid-Zufuhr wurde eine Probe entnommen und analysiert. Es wurde ein Restgehalt an Ethylenoxid unter 1 ppm und ein Dioxan-Gehalt von ungefähr 70 ppm bestimmt.

### Beispiel 3

Herstellung eines Polyoxyethylen-25-nonylphenolethers im Schleifenreaktor (Fig. 1)

In einen Schleifenreaktor nach Figur 1 werden 8,2 kg Nonylphenol mit 0,1 kg Na-Methylat als Katalysator mit einer Temperatur von 100°C eingefüllt, vermischt und intensiv getrocknet. Der Reaktor wird verschlossen, dreimal evakuiert und jeweils das Vakuum mit Stickstoff wieder aufgehoben. Die Temperatur wird unter Umwälzung auf 165°C erhöht.

Bei Erreichen dieser Temperatur werden 42 kg flüssiges Ethylenoxid sofort mit einer konstanten Zugaberate von 60 kg/Std. zugegeben. Es stellt sich ein Druck von 3,4 (bar zu Beginn ein, der sich dann aber im Verlauf der Reaktion auf 4,5 bar erhöht. Die Produkttemperatur steigt gleichzeitig auf 175 C° an. Nach Beendigung der Ethylenoxid-Zugabe fällt der Druck innerhalb einer Minute auf 1,5 bar.

10 Minuten nach Beendigung der Ethylenoxid-Zugabe wurde eine Probe entnommen und analysiert. Der Restgehalt an Ethylenoxid lag unter 1 ppm und an Dioxan bei 7 ppm.

### Beispiel 4

Herstellung von Polyoxyethylen-20-laurylether im Schleifenreaktor mit aussen angeordneter Strahlsauger-Mischdüse und einem innen angeordneten Düsenring (Fig. 2).

In einer Anlage gemäss Beispiel 1 und unter sonst gleichen Bedingungen und mit gleichen Stoffmengen wie in Beispiel 1 wurde in Abänderung der Versuchsbedingungen nach dem zweimaligen Spülen der Anlage vor Reaktionsbeginn mit Stickstoff beim dritten Aufheben des Vakuums der Stickstoffdruck in der Anlage auf 3,5 bar eingestellt.

Der Druck in der Anlage stieg von einem Anfangswert zu Beginn der Reaktion von ca. 4,9 bar auf einen Wert von ca. 10,6 bar zum Ende der Ethylenoxidzugabe. Nach Beendigung der Ethylenoxidzugabe fiel der Druck innerhalb von 2 Minuten auf ca. 9,2 bar zurück.

Die 10 Minuten nach Beendigung der Ethylenoxidzugabe entnommene Produktprobe enthielt einen Restgehalt an Ethylenoxid unter 1 ppm, während der Gehalt an Dioxan unter 3 ppm lag.

### Beispiel 5

Herstellung von Polyoxyethylen-3-laurylether (Fig. 2)

In eine Anlage gemäss Beispiel 1 wurden 28 kg Laurylalkohol gemäss Vergleichsbeispiel 1 und 0,15 kg Natriummethylat eingewogen, auf 100°C unter Umwälzung erhitzt und gleichzeitig intensiv gemischt. Durch Evakuieren unter Umwälzung und Beibehaltung der Temperatur wird das Reaktionsgemisch getrocknet. Sodann wird das Vakuum mit Stickstoff aufgehoben und noch zweimal evakuiert und jeweils das Vakuum mit Stickstoff wieder aufgehoben. Vor Beginn der Ethylenoxidzugabe wird der Stickstoffdruck auf 2 bar erhöht und die Temperatur im Reaktor auf 80°C gesenkt.

Nach Erreichen der Temperatur von 80°C wird sofort mit der Zugabe von Ethylenoxid mit einer Zugaberate von 10kg/h unter Beibehaltung der Temperatur zwischen 80 und 90°C begonnen. Es ist keine Induktionsperiode zu beobachten. Ohne Unterbrechung wird die Zugabe von Ethylenoxid bis zu einer Gesamtmenge von 19,5 kg fortgeführt. Nach Ende der Ethylenoxidzugabe wird die Temperatur kurzzeitig für 5 Minuten auf 130°C erhöht und sodann wieder auf 80°C gesenkt.

Der Druck im Reaktor betrug zu Beginn der Ethylenoxidzugabe 3 bar und erhöhte sich durch die weitere Zugabe auf 3,6 bar in der Endphase der Ethylenoxidzugabe. Nach Abkühlung auf 80°C betrug der Druck im Reaktor wiederum 2 bar.

Im Reaktionsprodukt, dem Polyoxyethylen-3-laurylether, war weder Ethylenoxid mit einer Nachweisempfindlichkeit von 0,2 ppm noch Dioxan mit einer Nachweisempfindlichkeit von 1 ppm nachzuweisen.

### Vergleichsbeispiel 6

Herstellung von Polyoxypropylen-15-laurylether

In einem Rührautoklaven gemäss Vergleichsbeispiel 4 wird 7,3 kg Laurylalkohol (Jodzahl <0,3) mit 40 g Natronlauge-Pulver (98%ig) vorgelegt und wie im Vergleichsbeispiel 4 behandelt, getrocknet, inertisiert und auf eine Temperatur von 125°C eingestellt.

Sodann werden 32,7 kg Propylenoxid durch das Einleitungsrohr unter Beachtung der maximalen Reaktonstemperatur von 140°C über einen Zeitraum von ca. 6 Stunden gleichmässig zudosiert.

Das erhaltene Reaktionsprodukt hatte eine Jodzahl von 5. Dieses deutet auf die Bildung von erheblichen Mengen ungesättigter Verbindungen als unerwünschte Nebenprodukte hin.

### Beispiel 6

Herstellung von Polyoxypropylen-15-laurylether

Abweichend von Vergleichsbeispiel 6 wurde obiges Produkt unter sonst gleichen Bedingungen in einer Anlage (Fig. 2) gemäss Beispiel 1 hergestellt.

Das erhaltene Reaktionsprodukt hatte eine Jodzahl von 0,5. Dieses bedeutet eine erhebliche Verbesserung der Reinheit gegenüber dem Produkt gemäss Vergleichsbeispiel 6.

## Patentansprüche

1. Verfahren zur sicheren und umweltschonenden Herstellung hochreiner Alkylenoxid-Addukte durch Umsetzung von Alkylenoxid mit einer organischen Verbindung mit wenigstens einem reaktiven Wasserstoffatom in einem Schleifenreaktor mit über eine Strahlsauger-Mischdüse gekoppeltem Gas- und Flüssigkeits-Kreislauf, dadurch gekennzeichnet, dass man die Temperatur der gesamten aus Alkylenoxid und gegebenenfalls zusätzlichem Inertgas bestehenden Gasphase im Gaskreislauf der Anlage während der Reaktion unterhalb der Temperatur der Flüssigphase hält.

2. Verfahren nach Anspruch 1, dadurch gekennzeichnet, dass die Herabsetzung der Temperatur der Gasphase gegenüber jener der Flüssigphase durch Entzug von Wärmeenergie mittels Verdampfung von flüssigem Alkylenoxid gewährleistet wird.

3. Verfahren nach Anspruch 1 und 2, dadurch gekennzeichnet, dass die Verdampfung des Alkylenoxides im Gasraum des Reaktors durch Feinverteilung des flüssigen Alkylenoxides, z.B. mittels eines Düsenringes, durchgeführt wird.

4. Verfahren nach Anspruch 1 und 2, dadurch gekennzeichnet, dass die Herabsetzung der Temperatur der Gasphase gegenüber jener der Flüssigphase ausserdem durch Entzug von Wärmeenergie über die die Gasphase berührenden und einschliessenden Anlagenwandungen gewährleistet wird.

5. Verfahren nach Anspruch 4, dadurch gekennzeichnet, dass man den Gaskreislauf durch eine ausserhalb des Reaktors geführte Verbindung zwischen dem Gasraum des Reaktors und dem Ansaugstutzen der Strahlsauger-Mischdüse aufrecht erhält und diese aussenliegende Verbindung selbst als Wärmeaustauscher ausbildet.

6. Verfahren nach einem der Ansprüche 1 bis 5, dadurch gekennzeichnet, dass als selbstansaugende Mischdüse ein Strahlsauger verwendet wird, dessen Treibstrahl eine Austrittsgeschwindigkeit von mindestens 10 m/sec aufweist.

7. Verfahren nach Anspruch 6, dadurch gekennzeichnet, dass als selbstansaugende Mischdüse ein Strahlsauger verwendet wird, dessen Treibstrahl eine Austrittsgeschwindigkeit von mindestens 20 m/sec aufweist.

8. Verfahren nach einem der Ansprüche 1 bis 7, dadurch gekennzeichnet, dass die Umwälzung der Flüssigphase so bemessen wird, dass der Reaktorinhalt zu jedem Zeitpunkt während der Reaktion mindestens 10mal pro Stunde umgewälzt wird.

9. Verfahren nach Anspruch 8, dadurch gekennzeichnet, dass die Umwälzung der Flüssigphase so bemessen wird, dass der Reaktorinhalt zu jedem Zeitpunkt während der Reaktion mindestens 30mal pro Stunde umgewälzt wird.

10. Verfahren nach einem der Ansprüche 1 bis 9, dadurch gekennzeichnet, dass der Inertgas-Partialdruck im Reaktor während der Reaktion auf mindestens 80% des Alkylenoxid-Partialdruckes gehalten wird.

11. Verfahren nach einem der Ansprüche 1 bis 9, dadurch gekennzeichnet, dass der Inertgas-Partialdruck im Reaktor während der Reaktion 1 bis 25 bar beträgt.

12. Verfahren nach Anspruch 11, dadurch gekennzeichnet, dass der Inertgas-Partialdruck im Reaktor während der Reaktion im Bereich von 3 bis 7 bar gehalten wird.

## Claims

1. A process for the safe and environmentally sound production of highly pure alkylene oxide adducts by reacting an alkylene oxide with an organic compound having at least one reactive hydrogen atom in a loop reactor with gas and liquid circulations coupled via an ejector mixing nozzle, characterized in that the temperature of the entire gas phase, consisting of alkylene oxide and, optionally, additional inert gas, in the gas circulation of the unit is maintained below the temperature of the liquid phase during the reaction.

2. The process according to claim 1, characterized in that the reduction of the temperature of the gas phase relative to that of the liquid phase is ensured by removing thermal energy by means of evaporation of the liquid alkylene oxide.

3. The process according to claims 1 and 2, characterized in that the evaporation of the alkylene oxide in the gas space of the reactor is effected by fine distribution of the liquid alkylene oxide, for example by means of a nozzle ring.

4. The process according to claims 1 and 2, characterized in that the reduction of the temperature of the gas phase relative to that of the liquid phase is additionally ensured by removing thermal energy via the plant walls which are in contact with and enclose the gas phase.

5. The process according to claim 4, characterized in that the gas circulation is maintained by a connection routed outside the reactor between the gas space of the reactor and the suction branch of the ejector mixing nozzle, and this outside connection itself is designed as a heat exchanger.

6. The process according to any of the claims 1 to 5, characterized in that an ejector whose driving jet has an exit velocity of at least 10 m/sec is used as the self-aspirating mixing nozzle.

7. The process according to claim 6, characterized in that an ejector whose driving jet has an exit velocity of at least 20 m/sec is used as the self-aspirating mixing nozzle.

8. The process according to any of claims 1 to 7, characterized in that the liquid phase is circulated at such a rate that the reactor contents are circulated at least 10 times per hour at all times during the reaction.

9. The process according to claim 8, characterized in that the liquid phase is circulated at such a rate that the reactor contents are circulated at least 30 times per hour at all times during the reaction.

10. The process according to any of claims 1 to 9, characterized in that the inert gas partial pressure in the reactor is maintained during the reaction at at least 80% of the alkylene oxide partial pressure.

11. The process according to any of claims 1 to 9, characterized in that the inert gas partial pressure in the reactor is 1 to 25 bar during the reaction.

12. The process according to claim 11, characterized in that the inert gas partial pressure in the reactor is maintained in the range from 3 to 7 bar during the reaction.

## Revendications

1. Procédé servant à préparer, de manière sûre et ménageant l'environnement, des produits d'addition de haute pureté d'oxyde d'alcoylène par réaction d'oxyde d'alcoylène avec un composé organique comportant au moins un atome d'hydrogène actif, dans un réacteur à circuit fermé avec une circulation de gaz et de liquide, couplées par l'intermédiaire d'une tuyère mélangeuse à jet d'aspiration, caractérisé en ce que, pendant la réaction, on maintient la température de la phase gazeuse entière, consistant en oxyde d'alcoylène et, cas échéant, un gaz inerte additionel, dans le circuit de gaz de l'installation, au-dessous de la température de la phase liquide.

2. Procédé selon la revendication 1, caractérisé en ce que l'abaissement de la température de la phase gazeuse par rapport à celle de la phase liquide est assuré par évacuation d'énergie calorique au moyen de l'évaporation d'oxyde d'alcoylène liquide.

3. Procédé selon la revendication 1 et 2, caractérisé en ce que l'évaporation de l'oxyde d'alcoylène dans l'espace de gaz du réacteur est effectuée par une répartition fine de l'oxyde d'alcoylène, par exemple, au moyen d'un anneau de tuyères.

4. Procédé selon la revendication 1 et 2, caractérisé en ce que l'abaissement de la température de la phase gazeuse par rapport à celle de la phase liquide est assuré, en outre, par évacuation d'énergie calorique à partir des parois de l'installation qui sont en contact avec la phase gazeuse et la renferment.

5. Procédé selon la revendication 4, caractérisé en ce que l'on entretient la circulation du gaz par une connection, réalisée à l'extérieur du réacteur, entre l'espace de gaz du réacteur et la tubulure d'aspiration de la tuyère mélangeuse à jet d'aspiration et que l'on construit cette connection externe elle-même comme un échangeur de chaleur.

6. Procédé selon l'une quelconque des revendications 1 à 5, caractérisé en ce qu'on utilise comme tuyère mélangeuse autoaspirante un jet d'aspiration dont le jet propulseur a une vitesse de sortie d'au moins 10 m/sec.

7. Procédé selon la revendication 6, caractérisé en ce qu'on utilise comme tuyère mélangeuse autoaspirante un jet d'aspiration dont le jet propulseur a une vitesse de sortie d'au moins 20 m/sec.

8. Procédé selon l'une quelconque des revendications 1 à 7, caractérisé en ce que la circulation de la phase liquide est réglée de manière telle que le contenu du réacteur passe, à chaque moment au cours de la réaction, au moins 10 fois par heure en circulation.

9. Procédé selon la revendication 8, caractérisé en ce que la circulation de la phase liquide est réglée de façon telle que le contenu du réacteur passe, à chaque moment au cours de la réaction, au moins 30 fois par heure en circulation.

10. Procédé selon l'une quelconque des revendications 1 à 9, caractérisé en ce que la pression partielle du gaz inerte dans le réacteur est maintenue pendant la réaction à au moins 80% de la pression partielle de l'oxyde d'alcoylène.

11. Procédé selon l'une quelconque des revendications 1 à 9, caractérisé en ce que la pression partielle du gaz inerte dans le réacteur est de 1 à 25 bar au cours de la réaction.

12. Procédé selon la revendication 11, caractérisé en ce que le pression partielle du gaz inerte dans le réacteur est maintenue dans le domaine de 3 à 7 bar au cours de la réaction.
